# EUROPEAN PATENT SPECIFICATION

(11) **EP 1 430 841 B1**
(45) Date of publication and mention of the grant of the patent: **30.04.2008**
(21) Application number: 03257978.1
(22) Date of filing: 18.12.2003
(51) Int. Cl.: A61B 17/15

(54) **Surgical instrument**
Chirurgisches Instrument
Instrument chirurgical

(30) Priority: 20.12.2002 US 325088
(43) Date of publication of application: 23.06.2004
(73) Proprietor: Zimmer Technology, Inc., Warsaw, IN 46580 (US)
(72) Inventor: Grimm, James E., Winona Lake Indiana 46804 (US); McGinley, Shawn E., Fort Wayne Indiana 46814 (US); Patmore, Donald M., Winona Lake Indiana 46590 (US)
(74) Representative: Mays, Julie

(56) References cited:
- WO-A-20/04017842
- WO-A-20/04019792
- FR-A- 2 776 176
- FR-A- 2 838 627
- US-A- 2 547 571
- US-A- 2 697 433
- US-A- 5 344 422
- US-A1- 2002 068 942

## Description

### 1. Field of the Invention.

The present invention relates to surgical instruments and, more specifically, to a surgical guide for properly positioning a surgical instrument with respect to an anatomical element.

### 2. Description of the Related Art.

The controlled positioning of surgical instruments is of significant importance in many surgical procedures and various methods and guide instruments have been developed for properly positioning a surgical instrument. Such methods include the use of surgical guides which function as mechanical guides for aligning drilling, cutting or milling instruments. The use of such surgical guides is common in orthopedic surgical procedures and such guides may be used to properly align a drill or cutting or milling instrument with respect to a bone when preparing the bone for receiving an implant such as an artificial joint. Computer assisted surgical procedures which involve the image guidance of a surgical instrument are also known. Image guidance techniques typically involve acquiring preoperative images of the relevant anatomical structures and generating a data base which represents a three dimensional model of the anatomical structures. The relevant surgical instruments typically have a known and fixed geometry which is also defined preoperatively. During the surgical procedure, the position of the instrument being used is registered with the anatomical coordinate system and a graphical display showing the relative positions of the tool and anatomical structure may be computed in real time and displayed for the surgeon to assist the surgeon in properly positioning and manipulating the surgical instrument with respect to the relevant anatomical structure.

In image guided procedures, a robotic arm may be used to position and control the instrument, or, the surgeon may manually position the instrument and use the display of the relative position of the instrument and anatomical structure when positioning the instrument.

Although, the known methods and instrumentation which are used to properly position surgical tools provide satisfactory results, the precision obtainable with image guided surgical systems often entails the use of expensive or cumbersome equipment may limit the use of such methods. FR-A-2 776 176 describes such a system. WO 2004/017842 and WO 2004/019792 fall under the incidence of Article 54(3) EPC and describe surgical instruments with an anchoring member which includes a spherical portion engageable with an instrument body.

### SUMMARY OF THE INVENTION

The present invention provides a surgical instrument that includes an anchoring member which is securable to an anatomical structure and which includes an instrument body which can be selectively repositioned relative to the anchoring member and anatomical structure after the anchoring member has been secured to the anatomical structure. A computer implemented image guidance system can be used to position the anchoring member when securing it to the anatomical structure and/or may also be used when adjusting the position of the instrument body relative to the anatomical structure after securement of the anchoring member to the anatomical structure. Such an instrument is useful in orthopedic surgical procedures for positioning a cutting or milling guide on a bone, such as a femur, for resection of the bone.

The invention comprises, in one form thereof, a surgical instrument for use with an anatomical structure. The instrument includes an anchoring member having a first portion securable to the anatomical structure, an instrument body adjustably repositionable relative to the anchoring member, and at least one reference element mountable to the instrument body. The at least one reference element is registerable in a computer implemented image guidance system.

In alternative embodiments, the at least one reference element may be at least three non-linearly positioned reference elements. The at least one reference element may be disposed on a reference member removably mountable on said instrument body. The at least one at least one reference element may also include at least three non-linearly positioned reference elements which are disposed on a reference member removably mountable on the instrument body. The reference element(s) may be a reflective structure or emit a signal. The instrument body may also define a positioning guide which is registerable with the reference member.

The first portion of the anchoring member may be a threaded shaft threadingly securable to a bone. The bone may be a femur and the instrument body may further include a positioning guide. The anchoring member may also define a first axis wherein the instrument body is adjustably pivotal about a second axis disposed substantially perpendicularly to the first axis. The anchoring member may also include a spherical portion engageable with the instrument body. The spherical portion may be seated within an adjustably pivotal bearing.

In one embodiment, the anchoring member includes a shaft defining a first axis and a spherical portion seated within an adjustably pivotal bearing. The bearing is pivotal about a second axis disposed substantially perpendicularly to the first axis. The instrument further includes first and second adjustment members. The first adjustment member is selectively engageable with the spherical portion wherein engagement of the first adjustment member secures the anchoring member relative to the instrument body in a selected rotational position with respect to the first axis. The second adjustment member is selectively engageable with the pivotal bearing wherein engagement of the second adjustment member secures the anchoring member relative to the instrument body in a selected rotational position with respect to the second axis.

The invention comprises, in another form thereof, a surgical instrument for use with an anatomical structure. The instrument includes an anchoring member having a shaft securable to the anatomical structure wherein the shaft defines a first axis. The instrument also includes an instrument body adjustably repositionable relative to the anchoring member wherein the instrument body is adjustably pivotal about a second axis disposed substantially perpendicular to the first axis. At least one reference element mountable on the instrument body is also provided. The at least one reference element is registerable in a computer implemented image guidance system wherein repositioning of the instrument body repositions the at least one reference element.

The surgical instrument may also include first and second adjustment members independently engageable with the instrument wherein engagement of the first adjustment member secures the anchoring member relative to the instrument body in a selected rotational position with respect to the first axis and engagement of the second adjustment member secures the anchoring member relative to the instrument body in a selected rotational position with respect to the second axis. The anchor member may also include a spherical portion seated within an adjustably pivotal bearing wherein the bearing is pivotal about the second axis and the second adjustment member selectively secures the bearing.

There is also described a method of positioning a surgical instrument with respect to an anatomical structure. The method includes providing an instrument having an anchoring member and an instrument body adjustably repositionable relative to said anchoring member. The method also includes securing the anchoring member to the anatomical structure and registering the position of the instrument body relative to the anatomical structure in a computer implemented image guidance system. Selectively adjusting the position of the instrument body relative to the anchoring member occurs after the steps of securing the anchoring member and registering the position of the instrument body.

The instrument body may be pivotal about first and second axes wherein the first axis is oriented substantially perpendicular to the second axis and the step of selectively adjusting the position of the instrument body includes securing the instrument body at selected rotational positions with respect to the first and second axes.

The step of registering the instrument body may include mounting at least one reference element detectable by the computer implemented guidance system on the instrument body and the at least one reference element may include three non-linearly positioned reference elements disposed on a reference member, the reference member being removably mountable on the instrument body.

The anatomical structure may be a femur wherein the anchoring member includes a shaft engageable with the femur and defining a first axis. The step of securing the anchoring member may include using the computer implemented image guidance system to secure the shaft substantially coaxially with the mechanical axis of the femur. The anchoring member may also include a spherical portion seated in a pivotal bearing wherein the bearing is pivotal about a second axis substantially perpendicular to the first axis. The instrument body may be secured at a selected rotational position with respect to the first axis by engaging the spherical portion with a first adjustment member and the instrument body may be secured at a selected rotational position with respect to the second axis by engaging the bearing with a second adjustment member.

There is also described a method of positioning a surgical instrument with respect to an anatomical structure. The method includes providing an instrument having an anchoring member and an instrument body adjustably repositionable relative to said anchoring member. The method also includes positioning the anchoring member relative to the anatomical structure using a computer implemented image guidance system and securing the anchoring member to the anatomical structure in a selected position. After securing the anchoring member to the anatomical structure, the position of the instrument body relative to the anchoring member is selectively adjusted.

The step of selectively adjusting the position of the instrument body may also include registering the position of the instrument body relative to the anatomical structure in the computer implemented image guidance system. Registering the position of the instrument body in the image guidance system may include mounting at least one reference element detectable by the computer implemented guidance system on the instrument body.

There is also described, a method of preparing a femur for an implant. An instrument having an anchoring member and an instrument body wherein the anchoring member has a shaft defining a first axis and the instrument body is adjustably repositionable relative to the anchoring member is provided. The method also includes securing the shaft of the anchoring member to the femur substantially coaxially with the mechanical axis of the femur with the aid of a computer implemented image guidance system. After securing the anchoring member to the femur, the position of the instrument body relative to the anchoring member is selectively adjusted.

The step of selectively adjusting the position of the instrument body may also include registering the position of the instrument body relative to the anatomical structure in the computer implemented image guidance system. Registering the position of the instrument body may include mounting at least one reference element detectable by the computer implemented guidance system on the instrument body.

The instrument body may be pivotal about the first axis and a second axis wherein the first axis is positioned substantially perpendicular to the second axis and the step of selectively adjusting the position of the instrument body includes securing the instrument body at selected rotational positions with respect to the first and second axes. The shaft may be a threaded shaft threadingly engagable with the femur. The anchoring member may also include a spherical portion seated in a pivotal bearing wherein the bearing is pivotal about the second axis and wherein securing the instrument body at a selected rotational position with respect to the first axis comprises engaging the spherical portion with a first adjustment member and securing the instrument body at a selected rotational position with respect to the second axis comprises engaging the bearing with a second adjustment member. The method may also include, after the step of selectively adjusting the position of the instrument body, the further step of registering a cutting or milling guide with the instrument body and securing the milling guide to the femur in a position defined by the registration of the milling guide with the instrument body.

The invention comprises, in another form thereof, a surgical instrument for use with an anatomical structure. The instrument includes an anchoring member having a first portion securable to the anatomical structure and an instrument body adjustably repositionable relative to said anchoring member. The instrument also includes at least two adjustment members wherein each of the adjustment members independently and positively secure the instrument body in a selected position with respect to one of six degrees of freedom. The six degrees of freedom are defined by translational movement along three substantially mutually perpendicular translational axes and rotational movement about three substantially mutually perpendicular rotational axes. At least one reference element mountable to said instrument body is also provided. The at least one reference element is registerable in a computer implemented image guidance system.

The at least two adjustment members may independently and positively secure the instrument body in a selected rotational position along one of two substantially perpendicular rotational axes, or, one of the adjustment members may independently and positively secure the instrument body in a selected translational position along a translational axis and one of the adjustment members may independently and positively secure the instrument body in a selected rotational position about a rotational axis.

The instrument may alternatively include at least three adjustment members wherein each of the adjustment members independently and positively secures the instrument body in a selected position with respect to one of the six degrees of freedom. One of the adjustment members may independently and positively secure the instrument body in a selected translational position along a translational axis and two of the adjustment members may independently and positively secure the instrument body in selected rotational positions about two substantially perpendicular rotational axes. The anchoring member may include a shaft and form one of the adjustment members wherein the anchoring member selectively secures the instrument body at a selected translational position along an axis defined by the shaft. Each of the adjustment members may include a threaded shaft.

There is also described a method of positioning a surgical instrument with respect to an anatomical structure. The method includes providing an instrument having an anchoring member and an instrument body adjustably repositionable relative to the anchoring member, securing the anchoring member to the anatomical structure and registering the position of the instrument body in a computer implemented image guidance system. The method also includes selectively adjusting the position of the instrument body relative to the anchoring member with regard to a first one of six degrees of freedom wherein the six degrees of freedom are defined by translational movement along three substantially perpendicular axes and rotational movement about three substantially perpendicular axes and selectively adjusting the position of said instrument body relative to the anchoring member with regard to a second one of the six degrees of freedom independently of the step of selectively adjusting the position of the instrument body relative to the anchoring member with regard to the first one of said six degrees of freedom. Each of the steps of selectively adjusting the position of the instrument body relative to the anchoring member occur after the steps of securing the anchoring member and registering the position of the instrument body.

The first one of the six degrees of freedom may define rotational movement about a first rotational axis and the second one of the six degrees of freedom may define rotational movement about a second rotational axis, the first and second rotational axes being substantially perpendicular, or, the first one of the six degrees of freedom may define rotational movement about a rotational axis and the second one of the six degrees of freedom may define translational movement along a translational axis. The method may also include, after the steps of securing the anchoring member and registering the position of the instrument body, the step of selectively adjusting the position of the instrument body relative to the anchoring member with regard to a third one of the six degrees of freedom independently of the steps of selectively adjusting the position of the instrument body relative to the anchoring member with regard to the first and second ones of the six degrees of freedom.

The step of registering the position of the instrument body includes mounting at least one reference element detectable by the computer implemented guidance system on the instrument body. The at least one reference element may include three non-linearly positioned reference elements disposed on a reference member wherein the reference member is removably mountable on the instrument body. The method may also include, prior to the step of securing the anchoring member to the anatomical structure, the step of positioning the anchoring member relative to the anatomical structure using the computer implemented image guidance system.

An advantage of the present invention is that, in some embodiments, it provides a surgical instrument which can be attached to an anatomical structure and adjustably repositioned relative to the anatomical structure after attachment wherein the adjustable repositioning of the instrument may be guided using a computer implemented image guidance system.

Another advantage of the present invention is that, in some embodiments, it provides a surgical instrument that has an anchoring member that can be secured to an anatomical structure using a computer implemented image guidance system and the instrument body can be adjustably repositioned, with or without the use of an image guidance system, after the anchoring member has been secured. When used to implant the femoral component of a prosthetic knee joint, this allows the anchoring member to be secured coaxially with the mechanical axis of the femur.

### BRIEF DESCRIPTION OF THE DRAWINGS

The above mentioned and other features and objects of this invention, and the manner of attaining them, will become more apparent and the invention itself will be better understood by reference to the following description of an embodiment of the invention taken in conjunction with the accompanying drawings, wherein:
Figure 1 is an exploded perspective view of a surgical instrument in accordance with the present invention.

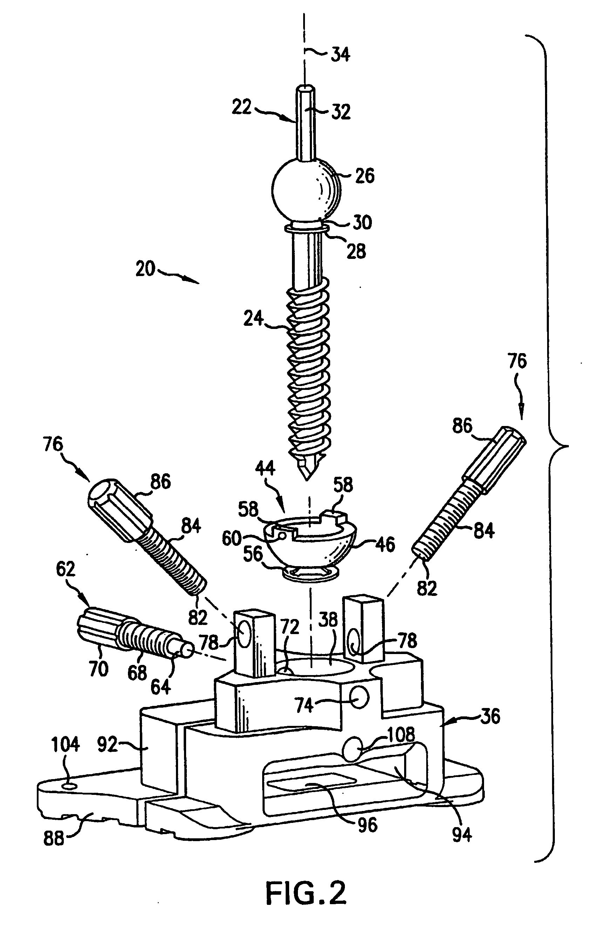

Figure 2 is another exploded perspective view of the surgical instrument of Figure 1.
Figure 3 is a top view of the surgical instrument of Figure 1.
Figure 4 is a view of a reference member having reference elements disposed thereon.
Figure 5 is a side view of the reference member of Figure 4.
Figure 6 is a view of a femur and tibia.
Figure 7 is a view of a base structure and cutting guide that can be used with the surgical instrument of Figure 1.

Corresponding reference characters indicate corresponding parts throughout the several views. Although the exemplification set out herein illustrates an embodiment of the invention, in one form, the embodiment disclosed below is not intended to be exhaustive or to be construed as limiting the scope of the invention to the precise form disclosed.

### DESCRIPTION OF THE PRESENT INVENTION

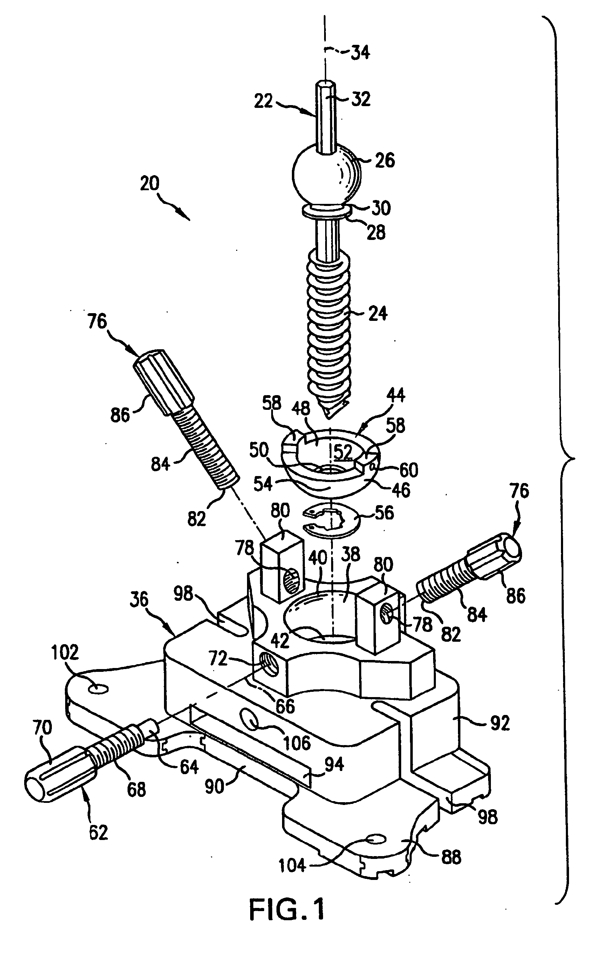

In accordance with the present invention, a surgical instrument 20 is shown in Figure 1. Instrument 20 includes an anchoring member 22. Anchoring member 22 has a first portion formed by threaded shaft 24 which is securable to an anatomical structure such as a bone. Shaft 24 has a configuration similar to the threaded shafts of conventional bone screws. Anchoring member 22 also includes a spherical portion 26. Located between the threads of threaded shaft 24 and spherical portion 26 is a collar 28 which defines an annular recess 30 between collar 28 and spherical portion 26. A hexagonal shaped shaft 32 is located coaxially with threaded shaft 24 on the opposite side of spherical portion 26. Hexagonal shaft 32 is engageable with a rotary driving device to rotate anchoring member 22 about axis 34 defined by shaft 24 and threadingly engage shaft 24 with an anatomical structure.

Anchoring member 24 is adjustably mounted on instrument body 36. Instrument body 36 defines a partially spherical recess 38 having oppositely disposed openings 40, 42. Opening 40 has a larger diameter than opening 42. Pivotal bearing 44 is mounted in recess 38. Bearing 44 includes a partially spherical shell portion 46 with oppositely disposed openings 48 and 50. Opening 48 has a larger diameter than opening 50. Spherical portion 26 of anchoring member 22 is seated within bearing 44 and bearingly contacts inner surface 52 of bearing 44. Outer surface 54 of bearing 44 bearingly contacts the surface of recess 38.

To assemble instrument 20, threaded shaft 24 is inserted through openings 48, 50 of bearing 44 and spherical portion 26 is retained within shell portion 46 by installing retaining clamp 56 in annular recess 30 to prevent shaft 24 from being retracted through opening 50. Bearing 44 includes tabs 58 having openings 60 and is pivotally mounted to instrument body 36 by inserting reduced diameter tips 64 of adjustment members 62 into openings 60. When mounted, bearing 44 pivots about axis 66 defined by adjustment members 62. Adjustment members 62 (only one is shown in the Figures) have a threaded portion 68 and a grip portion 70. Threaded portions 68 are engaged with threaded bores 72 and 74 in instrument body 36. When adjustment members 62 are relatively loosely tightened, bearing 44 is pivotal on tips 64. As one, or both, adjustment members 62 are tightened, bearing 44 becomes firmly engaged between adjustment members 62 and is thereby securable in a selected rotational position relative to axis 66.

Adjustment members 76 are mounted in threaded bores 78 located in projections 80 positioned adjacent recess 38 on instrument body 36. Threaded bores 78 are positioned at an angle relative to projections 80 so that distal ends 82 of adjustment members 76 are engageable with spherical portion 26 of anchoring member 22. Adjustment members 76 also include a threaded shaft 84 and a grip portion 86. Distal ends 82 of members 76 may form a portion of a sphere having the same radius of spherical portion 26 to provide a greater area of contact between distal ends 82 and spherical portion 26. When members 76 are relatively loosely tightened, spherical portion 26 may rotate relative to distal ends 82. Tightening members 76 firmly engages distal ends 82 with spherical portion 26 to secure anchoring member 22 in a selected position relative to instrument body 36.

Instrument body 36 includes a base portion 88 which includes a recess 90. Instrument body 36 also includes a central portion 92. A slot 94 is defined between base portion 88 and central portion 92. An opening 96 allows for the passage of threaded shaft 24 through base portion 88. Also defined by instrument body 36 are opposed slots 98 and bore holes 102, 104, 106 and 108.

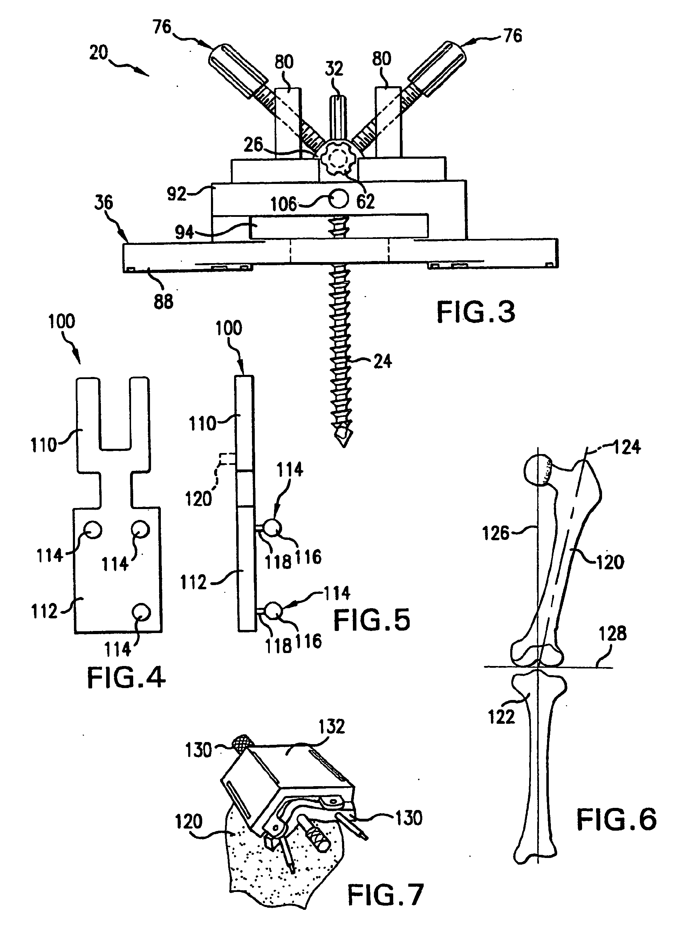

A reference member 100 is shown in Figures 4 and 5. Reference member includes a fork-shaped mounting portion 110 and a registration portion 112. Mounted on registration portion 112 are a plurality of reference elements 114. In the disclosed embodiment, three non-linearly positioned reference elements 114 are mounted on reference member 100 and have a spherical portion 116 mounted on a post 118. Spherical portion 116 is a reflective structure which is used to reflect light to facilitate the detection and registration of reference elements 114 in a computer implemented image guidance system as discussed in greater detail below.

Reference member 100 is removably mountable to instrument body 36 be positioning mounting portion 110 in slot 94. Mounting portion 110 is configured to closely fit slot 94 so that mounting of reference member 100 will position reference elements 114 at known relative positions and orientations to instrument body 36. Reference member 100 may optionally include a projection 120 extending transverse to the length of forked mounting portion 110 and which fits within recess 90 to facilitate the mounting of reference member 100 at a known and reproducible relative position to instrument body 36.

In alternative embodiments, reference elements 114 may be permanently secured to instrument body 36 or individually removably mounted to instrument body 36 such as to bore holes 102, 104, 106 and/or 108. Alternative reference elements may also include elements which emit a signal, such as an infrared emission, which is detectable by the computer implemented image guidance system or radio-opaque reference elements. If radio-opaque reference elements are employed, reference member 100 may be formed of a radio-transparent material and advantageously positions reference elements 114 at a distance from instrument body 36 which, in the illustrated embodiment is formed of stainless steel, a radio-opaque material which could interfere with the detection of radio-opaque reference elements positioned in close proximity to instrument body 36. In the illustrated embodiment, reference member 100 is an aluminum structure. The use of a removably mounted reference member 100 having reference elements 114 mounted thereon facilitates the use of instrument body 36 with different types of image guidance systems by allowing different reference members having the same physical shape but with different types of reference elements to be used with a single instrument body design.

The relevant dimensions of instrument 20 and the location of reference elements 114 relative to instrument body 36 when reference member 100 is mounted to instrument body 36 can be determined in advance and this data may be entered into an image guidance system. The relevant dimensional data concerning the anatomical structure which is the subject of the surgical procedure may also be entered into the image guidance system in advance of the surgical procedure.

As is known in the art, the relevant dimensional data concerning an anatomical structure of interest, e.g., a femur, may be determined using data acquired from images of the anatomical structure to generate a data base representing a model of the anatomical structure. The model of the anatomical structure may be a three dimensional model which is developed by acquiring a series of two dimensional images of the anatomical structure. Alternatively, the model of the anatomical structure may be a set of two dimensional images having known spatial relationships or other data structure which can be used to convey information concerning the three dimensional form of the anatomical structure. The model of the anatomical structure may then be used to generate displays of the anatomical structure from various perspectives for preoperative planning purposes and intraoperative navigational purposes. A variety of technologies which may be employed to generate such a model of an anatomical structure are well known in the art and include computed tomography (CT), magnetic resonance imaging (MRI), positron emission tomography (PET), ultrasound scanning and fluoroscopic imaging technologies.

The model of the anatomical structure obtained by such imaging technologies can be used for the intraoperative guidance of a surgical tool by facilitating the determination and display of the relative position and orientation of the surgical tool with respect to the actual anatomical structure. For example, if the model of the anatomical structure is a set of two dimensional images having known spatial relationships, several such images may be simultaneously displayed during the surgical procedure. By also displaying the position of the tool in the images and displaying images taken from different perspectives, e.g., one image facilitating the display of tool movement along the x and y coordinate axes and another image facilitating the display tool movement along the z axis, the individual images may together represent the movement of the tool in three dimensions.

For reference purposes, a coordinate system defined by the actual anatomical structure which is the subject of interest will be referred to herein as the anatomical coordinate system and a coordinate system defined by the model of the anatomical structure will be referred to as the image coordinate system. Data concerning the fixed size and shape of the surgical tool, or of a relevant portion thereof, which will be used in the image guided procedure is also determined pre-operativcly to obtain a three dimensional model of the tool or the relevant portions thereof.

Rigid anatomical structures, such as skeletal elements, are well suited for such image guided surgical techniques and individual skeletal elements may be used to define separate coordinate systems. The different rigid structures, e.g., skeletal elements, may be subject to relative movement, for example, the femur and tibia of a patient may be relatively moved during the surgical procedure and separate three dimensional models and coordinate systems may be created for the different skeletal elements. For example, during a knee replacement procedure, a three dimensional model of the tibia defining a first coordinate system may be utilized during the resection of the tibia while a separate coordinate system defined by a three dimension model of the femur is utilized during the resection of the femur.

When conducting image guided surgical techniques, the image coordinate system is registered with the anatomical coordinate system and the position of the surgical tool is also registered within the image coordinate system. After the registration of both the actual anatomical structure and the surgical tool, the relative position and orientation of the surgical tool may be communicated to the surgeon by displaying together images of the anatomical structure and tool based upon the three dimensional models of the anatomical structure and tool which were previously acquired.

Computer implemented image guidance systems which provide for the registration of an actual anatomical structure with a three dimensional model representing that structure together with the registration or localization of a surgical tool within the image coordinate system to facilitate the display of the relative positions of the surgical tool and the actual anatomical structure are known in the art. Known methods of registering the anatomical structure with the image coordinate system include the use of implanted fiducial markers which are recognizable by one or more scanning technologies. Alternatively, implants which may be located by physically positioning a digitizing probe or similar device in contact or at a known orientation with respect to the implant. Instead of using implants, it may also be possible to register the two coordinate systems by aligning anatomical landmark features.

Tracking devices employing various technologies enabling the registration or localization of a surgical tool and the tracking of the tool motion with respect to the anatomical coordinate system, which has been registered with the image coordinate system, are also known. For example, optical tracking systems which detect light from reflected or emitted by reflective targets or localizing emitters secured in a known orientation to the tool are known for determining the position of a surgical tool and registering the position of the tool within an image coordinate system representing a three dimensional model of an anatomical structure. For example, such a tracking system may take the form of a sensor unit having one or more lenses each focusing on separate charge coupled device (CCD) sensitive to infrared light. The sensor unit detects infrared light emitted by three or more non-linearly positioned light emitting diodes (LEDs) secured relative to the tool. A processor analyzes the images captured by the sensor unit and calculates the position and orientation of the tool. By registering the position of the sensing unit within the image coordinate system, the position of the tool relative to the anatomical structure, which has also been registered with the image coordinate system, may be determined and tracked as the tool is moved relative to the anatomical structure.

Alternative localizing systems may employ localizing emitters which emit an electromagnetic signal in the radio frequency or which emit visible light. It is also possible to employ digitizing physical probes which are brought into physical contact with the tool at predefined locations on the tool to register the position of the tool.

In the disclosed embodiment, the localizing system includes a light source and reference elements 114 reflect the light. The localizing system then detects the reflected light and computes the location of the individual reference elements 114 in a known manner. Reference elements 114 may be obtained from Northern Digital Inc. having a place of business at 103 Randall Dr., Waterloo, Onterio, Canada, N2V1C5. Other types of localizing systems may also be used with the present invention, such as those employing reflecting elements which emit a signal or which are radio-opaque. Known localizing systems of computer implemented image guidance systems may also be used to determine the relative position of an radio-opaque structure having an identifiable shape such as threaded shaft 24. Northern Digital Inc. supplies image guidance systems under the brand names Optotrak® and Polaris® which may be used with the present invention.

The use of instrument 20 in the resection of a distal femur will now be discussed. When implanting a prosthetic knee joint, the distal femur must be prepared to receive the femoral implant. The preparation of the distal femur typically involves resecting the distal femur to form several intersecting planar surfaces which conform to the interior surface of the selected femoral component. Figure 6 illustrates a femur 120 and tibia 122. The anatomical axis 124 of femur 120 is defined by the intramedullary canal of femur 120. The mechanical axis 126 of femur 120 extends from the center of the femoral head on the proximal femur to the center of the intercondylar notch on the distal femur. For many individuals the angle between the anatomical axis 124 and the mechanical axis 126 is approximately six degrees.

It is common to use the intramedullary canal of the femur as a reference structure when positioning a resection guide, such as a cutting or milling guide, on the distal femur to properly guide the milling or cutting instrumentation used to resect the distal femur. It is the position of the mechanical axis of the femur, however, which determines the best location of the resection planes to be formed on the distal femur. Thus, when using the intramedullary canal as a reference structure, the difference between the mechanical and anatomical axes of the femur must be addressed. Anchoring member 22 of the present invention, however, can be secured to femur 120 substantially coaxially with the mechanical axis 126 of femur 120.

When securing surgical instrument 20 to femur 120, reference member 100 is mounted to instrument body 36 and registered in the computer implemented image guidance system as described above. Similarly, femur 120 is registered within the image guidance system. The computer implemented image guidance system is then used to position anchoring member 22 coaxially with the mechanical axis of the femur. The location of the mechanical axis is determined preoperatively. For example, the surgeon may input the mechanical axis location into the image guidance system by indicating the location of two points on the mechanical axis on the images of the femur. It would also possible for the image guidance system to automatically determine the location of the mechanical axis using the model data representing the femur. Once placed in the selected position coaxially with the mechanical axis, anchoring member 22 is secured to femur 120. When positioning anchoring member 22 relative to femur 120, anchoring member 22 may be placed in a default position relative to body 36 and reference elements 114 mounted on instrument body 36 used to track the position of anchoring member 22 relative to femur 120. Alternatively, anchoring member 22 may be directly detected and tracked by the image guidance system.

After anchoring member 22 has been secured to femur 120, the image guidance system is used to determine if instrument body 36 is in the desired position relative to femur 120. The desired position of instrument body 36 is determined preoperatively and input into the image guidance system. Instrument body 36 is then adjusted relative to anchoring member 22 and femur 120 to align instrument 36 with its desired position. First, instrument body 36 is pivoted about axis 66 of pivotal bearing 44 to obtain the desired varus/valgus alignment, e.g., parallel to the transverse axis 128. When instrument body 36 is in the desired varus/valgus orientation, adjustment members 62 are tightened to prevent pivotal motion of bearing 44. Next, the desired "external rotation" of the instrument body 36 is checked using the image guidance system and adjusted if necessary. The external rotation of instrument body 36 refers to the rotational orientation of instrument body 36 relative to axis 34 defined by anchoring member 22 which is positioned coaxially with the mechanical axis 126 of femur 120. After positioning instrument body 36 in the desired rotational position relative to axis 34, adjustment members 76 are firmly engaged with spherical portion 26 to secure instrument body 36 in the desired position relative to anchoring member 22. Anchoring member 24 is then rotated into, or out of, femur 120 to set the "depth" of the resection on the distal femur.

Instrument body 36 is thus adjustable with respect to three degrees of freedom after securing anchoring member 22 with a femur, i.e., instrument body 36 may be rotated about axis 66 and secured in a selected rotational position about axis 66 by tightly engaging adjustment members 62 with pivotal bearing 44; instrument body 36 may be rotated about axis 34 and secured in a selected rotational position about axis 34, which is substantially perpendicular to axis 66, by tightly engaging adjustment members 76 with spherical portion 26; and after initially securing anchoring member 22 to femur 120, instrument body 36 may be translated along axis 34 and placed in a selected position along axis 34 by rotating a translational adjustment member, i.e., anchoring member 22, further into, or out of, femur 120. Although the illustrated embodiment utilizes two adjustment members 62 to positively secure instrument body 36 in a selected rotational position about axis 66 and two adjustment members 76 to positively secure instrument body 36 in a selected rotational position about axis 34, alternative embodiments could employ two individual adjustment members to independently and positively secure instrument body 36 in selected rotational positions about axes 66 and 34.

With regard to the remaining three degrees of freedom, by maintaining relatively tight tolerances between anchoring member 22 and its interfaces with shell portion 46 and opening 50 in bearing 44 and clamp 56, anchoring member 22 can be prevented from pivoting about an axis which is substantially perpendicular to both axes 34 and 66. Rotation about this third axis and the translational position of instrument body 36 along this third axis and axis 66 are all determined by the position and orientation at which anchoring member 22 is engaged with the anatomical structure, e.g., femur 120. Alternative embodiments of the invention allowing the selective adjustment of instrument body 36 relative to anchoring member 22 along one or more of these three remaining degrees of freedom are also possible. The six degrees of freedom referred to herein are defined by translational movement about three substantially mutually perpendicular translational axes and rotational movement about three substantially mutually perpendicular rotational axes and thereby define translational coordinate system and a rotational coordinate system. The translational and rotational axes may be parallel or coincide, however, it is not necessary for such axes to be parallel or coincide.

After being positioned in the desired orientation on the distal femur, instrument body 36 may be used to position base structures 130 on the lateral and medial sides of the distal femur. Surgical instrument 20 may then be removed from the distal femur, a cutting guide 132 secured to base structures 130 as shown in Figure 7 and the distal femur resected with a cutting blade inserted through the various cutting slots defined by cutting guide 132. Femoral bases and cutting guides which may be used with surgical instrument 20 are available under the name 5-in-1 from Zimmer Inc. of Warsaw, Indiana and are described in U.S. Pat. No. 5,743,915 which is hereby incorporated herein by reference. Base structures 130 are positioned on the distal femur by placing base structures 130 into registering contacct with instrument body 36 and then securing base structures 130 directly to femur 120. Recess 90, slot 94, openings 102, 104, 106 or 108, slots 98 or other predefined surfaces on instrument body 36 may be used to register a base structure to properly position the base structure on the femur. Alternatively, an intermediate part may be removeably secured to instrument body 36, such as by insertion into a slot or opening on instrument body 36 and the base structure registered with the intermediate part. A cutting or milling guide or other surgical implement could also be formed directly on instrument body 36. Milling and cutting instrumentation which could be adapted for use with an instrument body 36 is disclosed in U.S. Pat. Nos. 5,474,559 and 5,593,411.

When implanting a prosthetic knee joint using instrument 20, a selectively adjustable surgical instrument that may be used to resect the tibia is described by James E. Grimm in a U.S. Patent Application US 2004 172044 entitled Surgical Instrument And Positioning Method having an attorney docket number of ZIM0164 and filed on the same date as the present application .

While this invention has been described as having an exemplary design, the present invention may be further modified.

## Claims

1. A surgical instrument (20) for use with an anatomical structure, said instrument (20) comprising:
an anchoring member (22) having a first portion(24) securable to the anatomical structure;
an instrument body (36) adjustably repositionable relative to said anchoring member (22); and
at least one reference element (100) mountable to said instrument body (36), said at least one reference element (100) registerable in a computer implemented image guidance system **characterized in that** said anchoring member (22) further includes a spherical portion (26) engageable with said instrument body said surgical instrument further comprising first and second adjustment members independently engageable with said instrument, engagement of said first adjustment member (64) securing said anchoring member (22) relative to said instrument body (36) in a selected rotational position With respect to a first axis, engagement of said second adjustment member securing said anchoring member relative to said instrument body (36) in a selected rotational position with respect to a second axis.

2. The surgical instrument of claim 1 **characterized in that** said at least one reference element (100) comprises at least three non-linearly positioned reference elements (114).

3. The surgical instrument of Claim 1 **characterized in that** said at least one reference element (100) is removably mountable on said instrument body (3 6).

4. The surgical instrument of Claim 1 **characterized in that** said at least one reference element includes at least three non-linearly positioned reference elements (114) disposed on a reference member removably mountable on said instrument body (36).

5. The surgical instrument of Claim 4 **characterized in that** said instrument body (36) defines a positioning guide and said reference member (100) is registerable with said positioning guide.

6. The surgical instrument of Claim 1 **characterized in that** said first portion (24) of said anchoring member (22) is a threaded shaft threadingly securable to a bone.

7. The surgical instrument of Claim 1 **characterized in that** said anchoring member (22) defines a first axis and said instrument body (36) is adjustably pivotal about a second axis disposed substantially perpendicularly to said first axis.

8. The surgical instrument of Claim 7 **characterized in that** said first portion of said anchoring member (24) is a threaded shaft threadingly securable to a bone.

9. The surgical instrument of Claim 1 **characterized in that** said spherical portion (26) is seated within an adjustably pivotal bearing(44), said bearing (44) pivotal about a second axis.

10. The surgical instrument of Claim 1 **characterized in that** said anchoring member (22) includes a shaft (24) defining a first axis and said spherical portion (26) seated within an adjustably pivotal bearing (44), said bearing (44) pivotal about a second axis disposed substantially perpendicularly to said first axis, and said instrument further includes first and second adjustment members, (62, 76) said first adjustment member (62) selectively engageable with said spherical portion wherein engagement of said first adjustment member (64) secures said anchoring member (22) relative to said instrument body (36) in a selected rotational position with respect to said first axis; said second adjustment member (76) selectively engageable with said pivotal bearing (44) wherein engagement of said second adjustment member (76) secures said anchoring member relative to said instrument body (36) in a selected rotational position with respect to said second axis.

11. The surgical instrument of Claim 1**characterized in that** said first portion of said anchoring member (22) is a threaded shaft threadingly securable to a femur and said instrument body (36) further comprises a positioning guide.

12. The surgical instrument of Claim 1**characterized in that** said at least one reference element (114) is a reflective structure.

13. The surgical instrument of Claim 1 **characterized in that** said at least one reference element (114) emits a signal.

14. The surgical instrument of Claim I **characterized in that** repositioning of said instrument body (36) repositions said at least one reference element (114).

15. The surgical instrument of Claim 1 **characterized in that** said anchor member (22) further comprises said spherical portion (26) seated within an adjustably pivotal bearing(44), said bearing (44) pivotal about said second axis and said second adjustment member selectively securing said bearing (44).

16. The surgical instrument (20) of claim 14 **characterized in that** said anchoring member (22) further includes said spherical portion (26), said spherical portion disposed within a bearing adjustably pivotal about said second axis.

17. The surgical instrument of Claim 14 **characterized in that** said at least one reference element includes at least three non-linearly positioned reference elements (114).

18. The surreal instrument of Claim 14 **characterised in** mat said at least one reference element includes at least three non-linearly positioned reference elements (114) disposed on a reference member (100), said reference member (100) being removably mountable on said instrument body (36).

19. The surgical instrument of Claim 14 **characterized in that** said at least one reference element (114) is a reflective structure.

20. The surgical instrument of Claim 14 **characterized in that** said at least one reference element (114) emits a signal.

## Patentansprüche

1. Chirurgisches Instrument (20) zur Verwendung mit einer anatomischen Struktur, wobei das Instrument (20) aufweist:
ein Verankerungselement (22), das einen ersten Abschnitt (24) aufweist, der an der anatomischen Struktur sicherbar ist;
einen Instrumentenkörper (36), der relativ zum Verankerungselement (22) verstellbar umpositionierbar ist; und
mindestens ein Bezugselement (100), das am Instrumentenkörper (36) anbringbar ist, wobei das mindestens eine Bezugselement (100) in einem computerimplementierten Bildführungssystem registrierbar ist, **dadurch gekennzeichnet, daß** das Verankerungselement (22) ferner einen sphärischen Abschnitt (26) aufweist, der mit dem Instrumentenkörper in Eingriff bringbar ist, wobei das chirurgische Instrument ferner erste und zweite Verstellelemente aufweist, die unabhängig mit dem Instrument in Eingriff bringbar sind, wobei der Eingriff des ersten Verstellelements (64) das Verankerungselement (22) relativ zum Instrumentenkörper (36) in einer ausgewählten Rotationsposition bezüglich der ersten Achse sichert, und der Eingriff des zweiten Verstellelement das Verankerungselement relativ zum Instrumentenkörper (36) in einer ausgewählten Rotationsposition bezüglich der zweiten Achse sichert.

2. Chirurgisches Instrument nach Anspruch 1, **dadurch gekennzeichnet, daß** das mindestens eine Bezugselement (100) mindestens drei nicht-linear positionierte Bezugselemente (114) aufweist.

3. Chirurgisches Instrument nach Anspruch 1, **dadurch gekennzeichnet, daß** das mindestens eine Bezugselement (100) abnehmbar am Instrumentenkörper (36) anbringbar ist.

4. Chirurgisches Instrument nach Anspruch 1, **dadurch gekennzeichnet, daß** das mindestens eine Bezugselement mindestens drei nicht-linear positionierte Bezugselemente (114) aufweist, die an einem Bezugselement angeordnet sind, das am Instrumentenkörper (36) abnehmbar anbringbar ist.

5. Chirurgisches Instrument nach Anspruch 4, **dadurch gekennzeichnet, daß** der Instrumentenkörper (36) eine Positionierungsführung definiert und das Bezugselement (100) mit der Positionierungsführung registrierbar ist.

6. Chirurgisches Instrument nach Anspruch 1, **dadurch gekennzeichnet, daß** der erste Abschnitt (24) des Verankerungselements (22) ein Gewindeschaft ist, der durch Gewindeschneiden an einem Knochen sicherbar ist.

7. Chirurgisches Instrument nach Anspruch 1, **dadurch gekennzeichnet, daß** das Verankerungselement (22) eine erste Achse definiert und der Instrumentenkörper (36) drehbar um eine zweite Achse verstellbar ist, die im wesentlichen senkrecht zur ersten Achse angeordnet ist.

8. Chirurgisches Instrument nach Anspruch 7, **dadurch gekennzeichnet, daß** der erste Abschnitt des Verankerungselements (24) ein Gewindeschaft ist, der durch Gewindeschneiden an einem Knochen sicherbar ist.

9. Chirurgisches Instrument nach Anspruch 1, **dadurch gekennzeichnet, daß** der sphärische Abschnitt (26) in einem verstellbar drehbaren Lager (44) sitzt, wobei das Lager (44) um eine zweite Achse drehbar ist.

10. Chirurgisches Instrument nach Anspruch 1, **dadurch gekennzeichnet, daß** das Verankerungselement (22) einen Schaft (24) aufweist, der eine erste Achse definiert, und der sphärische Abschnitt (26) in einem verstellbar drehbaren Lager (44) sitzt, wobei das Lager (44) um eine zweite Achse drehbar ist, die im wesentlichen senkrecht zur ersten Achse angeordnet ist, und das Instrument ferner erste und zweite Verstellelemente (62, 76) aufweist, wobei das erste Verstellelement (62) selektiv mit dem sphärischen Abschnitt in Eingriff bringbar ist, wobei der Eingriff des ersten Verstellelements (64) das Verankerungselement (22) relativ zum Instrumentenkörper (36) in einer ausgewählten Rotationsposition bezüglich der ersten Achse sichert; das zweite Verstellelement (76) selektiv mit dem drehbaren Lager (44) in Eingriff bringbar ist, wobei der Eingriff des zweiten Verstellelements (76) das Verankerungselement relativ zum Instrumentenkörper (36) in einer ausgewählten Rotationsposition bezüglich der zweiten Achse sichert.

11. Chirurgisches Instrument nach Anspruch 1, **dadurch gekennzeichnet, daß** der erste Abschnitt des Verankerungselements (22) ein Gewindeschaft ist, der durch Gewindeschneiden an einem Femur sicherbar ist, und der Instrumentenkörper (36) ferner eine Positionierungsführung aufweist.

12. Chirurgisches Instrument nach Anspruch 1, **dadurch gekennzeichnet, daß** das mindestens eine Bezugselement (114) eine reflektierende Struktur ist.

13. Chirurgisches Instrument nach Anspruch 1, **dadurch gekennzeichnet, daß** das mindestens eine Bezugselement (114) ein Signal emittiert.

14. Chirurgisches Instrument nach Anspruch 1, **dadurch gekennzeichnet, daß** eine Umpositionierung des Instrumentenkörpers (36) das mindestens eine Bezugselement (114) umpositioniert.

15. Chirurgisches Instrument nach Anspruch 1, **dadurch gekennzeichnet, daß** das Verankerungselement (22) ferner den sphärischen Abschnitt (26) aufweist, der in einem verstellbar drehbaren Lager (44) sitzt, wobei das Lager (44) um die zweite Achse drehbar ist und das zweite Verstellelement selektiv das Lager (44) sichert.

16. Chirurgisches Instrument (20) nach Anspruch 14, **dadurch gekennzeichnet, daß** das Verankerungselement (22) ferner den sphärischen Abschnitt (26) aufweist, wobei der sphärische Abschnitt in einem Lager angeordnet ist, das drehbar um die zweite Achse verstellbar ist.

17. Chirurgisches Instrument nach Anspruch 14, **dadurch gekennzeichnet, daß** das mindestens eine Bezugselement mindestens drei nicht-linear positionierte Bezugselemente (114) aufweist.

18. Chirurgisches Instrument nach Anspruch 14, **dadurch gekennzeichnet, daß** das mindestens eine Bezugselement mindestens drei nicht-linear positionierte Bezugselemente (114) aufweist, die an einem Bezugselement (100) angeordnet sind, wobei das Bezugselement (100) abnehmbar am Instrumentenkörper (36) anbringbar ist.

19. Chirurgisches Instrument nach Anspruch 14, **dadurch gekennzeichnet, daß** das mindestens eine Bezugselement (114) eine reflektierende Struktur ist.

20. Chirurgisches Instrument nach Anspruch 1, **dadurch gekennzeichnet, daß** das mindestens eine Bezugselement (114) ein Signal emittiert.

## Revendications

1. Instrument chirurgical (20) à utiliser avec une structure anatomique, ledit instrument (20) comprenant:
un élément d'ancrage (22) ayant une première portion (24) pouvant être fixée à la structure anatomique;
un corps d'instrument (36) pouvant être repositionné de manière ajustable par rapport audit élément d'ancrage (22); et
au moins un élément de référence (100) pouvant être monté sur ledit corps d'instrument (36), ledit au moins un élément de référence (100) pouvant être enregistré dans un système de guidage par imagerie mis en oeuvre par ordinateur **caractérisé en ce que** ledit élément d'ancrage (22) inclut en plus une portion sphérique (26) pouvant être engagée avec ledit corps d'instrument ledit instrument chirurgical comprenant en plus un premier et un deuxième éléments d'ajustement pouvant être engagés de manière indépendante avec ledit instrument, l'engagement dudit premier élément d'ajustement (64) fixant ledit élément d'ancrage (22) par rapport audit corps d'instrument (36) dans une position de rotation sélectionnée par rapport audit premier axe, l'engagement dudit deuxième élément d'ajustement fixant ledit élément d'ancrage par rapport audit corps d'instrument (36) dans une position de rotation sélectionnée par rapport audit deuxième axe.

2. Instrument chirurgical de la revendication 1 **caractérisé en ce que** ledit au moins un élément de référence (100) comprend au moins trois éléments de référence positionnés de manière non linéaire (114).

3. Instrument chirurgical de la revendication 1 **caractérisé en ce que** ledit au moins un élément de référence (100) peut être monté de manière amovible sur ledit corps d'instrument (36).

4. Instrument chirurgical de la revendication 1 **caractérisé en ce que** ledit au moins un élément de référence inclut au moins trois éléments de référence positionnés de manière non linéaire (114) disposés sur un élément de référence pouvant être monté de manière amovible sur ledit corps d'instrument (36).

5. Instrument chirurgical de la revendication 4 **caractérisé en ce que** ledit corps d'instrument (36) définit un guide de positionnement et ledit élément de référence (100) peut être enregistré avec ledit guide de positionnement.

6. Instrument chirurgical de la revendication 1 **caractérisé en ce que** ladite première portion (24) dudit élément d'ancrage (22) est un arbre fileté pouvant être fixé par vissage à un os.

7. Instrument chirurgical de la revendication 1 **caractérisé en ce que** ledit élément d'ancrage (22) définit un premier axe et ledit corps d'instrument (36) pivote de manière ajustable autour d'un deuxième axe disposé de manière essentiellement perpendiculaire audit premier axe.

8. Instrument chirurgical de la revendication 7 **caractérisé en ce que** ladite première portion dudit élément d'ancrage (24) est un arbre fileté pouvant être fixé par vissage à un os.

9. Instrument chirurgical de la revendication 1 **caractérisé en ce que** ladite portion sphérique (26) siège dans un palier pivotant de manière ajustable (44), ledit palier (44) pivotant autour d'un deuxième axe.

10. Instrument chirurgical de la revendication 1 **caractérisé en ce que** ledit élément d'ancrage (22) inclut un arbre (24) définissant un premier axe et ladite portion sphérique (26) siégeant dans un palier pivotant de manière ajustable (44), ledit palier (44) pivotant autour d'un deuxième axe disposé essentiellement perpendiculairement audit premier axe, et ledit instrument inclut en plus un premier et un deuxième éléments d'ajustement (62, 76), ledit premier élément d'ajustement (62) pouvant être engagé de manière sélective avec ladite portion sphérique où l'engagement dudit premier élément d'ajustement (64) fixe ledit élément d'ancrage (22) par rapport audit corps d'instrument (36) dans une position de rotation sélectionnée par rapport audit premier axe; ledit deuxième élément d'ajustement (76) pouvant être engagé de manière sélective avec ledit palier pivotant (44) où l'engagement dudit deuxième élément d'ajustement (76) fixe ledit élément d'ancrage par rapport audit corps d'instrument (36) dans une position de rotation sélectionnée par rapport audit deuxième axe.

11. Instrument chirurgical de la revendication 1 **caractérisé en ce que** ladite première portion dudit élément d'ancrage (22) est un arbre fileté pouvant être fixé par vissage à un fémur et ledit corps d'instrument (36) comprend en plus un guide de positionnement.

12. Instrument chirurgical de la revendication 1 **caractérisé en ce que** ledit au moins un élément de référence (114) est une structure réfléchissante.

13. Instrument chirurgical de la revendication 1 **caractérisé en ce que** ledit au moins un élément de référence (114) émet un signal.

14. Instrument chirurgical de la revendication 1 **caractérisé en ce qu'**un repositionnement dudit corps d'instrument (36) repositionne ledit au moins un élément de référence (114).

15. Instrument chirurgical de la revendication 1 **caractérisé en ce que** ledit élément d'ancrage (22) comprend en plus ladite portion sphérique (26) siégeant dans un palier pivotant de manière ajustable (44), ledit palier (44) pivotant autour dudit deuxième axe et ledit deuxième élément d'ajustement fixant de manière sélective ledit palier (44).

16. Instrument chirurgical (20) de la revendication 14 **caractérisé en ce que** ledit élément d'ancrage (22) inclut en plus ladite portion sphérique (26), ladite portion sphérique disposée dans un palier pivotant de manière ajustable autour dudit deuxième axe.

17. Instrument chirurgical de la revendication 14 **caractérisé en ce que** ledit au moins un élément de référence inclut au moins trois éléments de référence positionnés de manière non linéaire (114).

18. Instrument chirurgical de la revendication 14 **caractérisé en ce que** ledit au moins un élément de référence inclut au moins trois éléments de référence positionnés de manière non linéaire (114) disposés sur un organe de référence (100), ledit organe de référence (100) pouvant être monté de manière amovible sur ledit corps d'instrument (36).

19. Instrument chirurgical de la revendication 14 **caractérisé en ce que** ledit au moins un élément de référence (114) est une structure réfléchissante.

20. Instrument chirurgical de la revendication l4**caractérisé en ce que** ledit au moins un élément de référence (114) émet un signal.
